# EUROPEAN PATENT APPLICATION

(11) **EP 3 747 449 A1**
(43) Date of publication of application: **09.12.2020**
(21) Application number: 19748455.3
(22) Date of filing: 07.02.2019
(51) Int. Cl.: A61K 35/12, A61K 35/34, A61K 35/52, A61K 35/28, A61P 19/02

(54) **PHARMACEUTICAL COMPOSITION COMPRISING ISOLATED MITOCHONDRIA FOR PREVENTION OR TREATMENT OF RHEUMATOID ARTHRITIS**

(30) Priority: 02.02.2018 KR 20180013278
(71) Applicant: Paean Biotechnology Inc., Daejeon 34028 (KR)
(72) Inventor: CHOI, Yong-Soo, Gunpo-si, Gyeonggi-do 15888 (KR); HWANG, Jung Uk, Geumnam-myeon, Sejong 30082 (KR); HAN, Kyuboem, Daejeon 34119 (KR); KIM, Chun-Hyung, Sejong 30130 (KR)
(74) Representative: HGF
(86) International application number: PCT/KR2019/001501
(87) International publication number: WO 2019/151840

(57) **Abstract**

The present invention relates to a pharmaceutical composition for prevention or treatment of rheumatoid arthritis and, more particularly, to a pharmaceutical composition compsiring mitochondria as an effective ingredient for prevention or treatment of rheumatoid arthritis. When a foreign mitochondria pharmaceutical composition of the present invention is administered to a subject suffering from rheumatoid arthritis, the subject may alleviate edema and rubefaction symptoms. In addition, the pharmaceutical composition of the present invention reduces an expression level of the inflammatory cytokine IL-6, but increases an expression level of the anti-inflammatory cytokine IL-10 in the subject. Therefore, the pharmaceutical composition according to the present invention can be effectively used for preventing or treating rheumatoid arthritis.

## Description

### TECHNICAL FIELD

The present invention relates to a pharmaceutical composition for preventing or treating rheumatoid arthritis, and more particularly, to a pharmaceutical composition, for preventing or treating rheumatoid arthritis, containing mitochondria as an active ingredient.

### BACKGROUND ART

Rheumatoid arthritis is a chronic inflammatory disease that proliferates synovial membrane of joints and destroys bone or cartilage. In the initial stage, inflammation occurs in the synovial membrane that lines the joint, and the inflammation gradually spreads across the surrounding cartilages and bones, resulting in destruction and deformation of the joint. In addition, rheumatoid arthritis may cause inflammatory response in various organs as well as joints, and may show symptoms such as subcutaneous nodule, pulmonary fibrosis, vasculitis, skin ulcers, rash, weight loss, etc. The exact cause of rheumatoid arthritis has not been found yet, and autoimmune response has been known as a major mechanism.

For treatment of rheumatoid arthritis, non-steroidal anti-inflammatory drugs, steroids, anti-rheumatic drugs, and tumor necrosis factor inhibitors have been used. The non-steroidal anti-inflammatory drugs and steroids relieve inflammation to alleviate symptoms of diseases, but does not suppress the progression of diseases. The anti-rheumatic drugs require a predetermined time taken to exhibit an effect compared to the non-steroidal anti-inflammatory drugs or steroids, and have little direct analgesic effect; however, the anti-rheumatic drugs have been used to fundamentally improve disease. Currently, for treatment of rheumatoid arthritis, the anti-rheumatic drugs are used together with the non-steroidal anti-inflammatory drugs or steroids until the effects of the anti-rheumatic drugs are obtained.

However, before the effects of the anti-rheumatic drugs are obtained, side effects such as gastrointestinal disorders may occur due to the use of non-steroidal anti-inflammatory drugs, and side effects such as reduction of immunity, increase of tolerance, and complications may occur due to the use of steroids. In addition, the anti-rheumatic drugs focuse on treatment of arthritis but may cause various side effects. Thus, follow-up observation including a periodic blood test is required.

In addition, tumor necrosis factor inhibitors have been prescribed for rheumatoid arthritis patients who do not respond to the anti-rheumatic drugs. The tumor necrosis factor inhibitors are therapeutic agents for suppressing an inflammatory response by blocking a tumor necrosis factor, which is a mediator causing rheumatoid arthritis. The tumor necrosis factor inhibitors are advantageous in that symptoms are alleviated by 70% or more in rheumatoid arthritis that does not respond to conventional anti-rheumatic drugs and the effects thereof are obtained faster than conventional therapeutic agents. However, there is a disadvantage that the tumor necrosis factor inhibitors have a short half-life and is not a therapeutic agent curing for fundamental causes of the disease. In addition, side effects of the tumor necrosis factor inhibitors such as activation of latent tuberculosis have been reported.

Meanwhile, mitochondria are cell organelles of eukaryotic cells, which are involved in the synthesis and regulation of adenosine triphosphate (ATP) that is an intracellular energy source. Mitochondria are associated with various metabolic pathways in the living body, such as cell signal processing, cell differentiation, cell death, as well as control of cell cycle and cell growth. With respect to rheumatoid arthritis, studies have been reported that synovial membrane proliferation of joints causes mitochondrial dysfunction and the mitochondrial dysfunction increases the production of reactive oxygen species to actively induce inflammatory responses (Fearon et al., Nat Rev Rheomatoid., 2016, (12): 385-397).

### DISCLOSURE OF THE INVENTION

### TECHNICAL PROBLEM

Studies have been conducted to treat rheumatoid arthritis, but there are problems such as a limited therapeutic range or occurence of side effects, and an innovative therapy has not been developed yet. Continuous research and development for safe and effective rheumatoid arthritis therapeutic agents are required.

Therefore, the object of the present invention is to provide a pharmaceutical composition for treating rheumatoid arthritis and a method for treating rheumatoid arthritis using the same.

### TECHNICAL SOLUTION

To solve the problem, the present invention provides a pharmaceutical composition for preventing or treating rheumatoid arthritis, the pharmaceutical composition containing mitochondria as an active ingredient.

The present invention also provides a method for preventing or treating rheumatoid arthritis, the method including administering the pharmaceutical composition to an individual.

### ADVANTAGEOUS EFFECTS

When a pharmaceutical composition containing exogenous mitochondria according to the present invention is administered to an individual who is suffering from rheumatoid arthritis, swelling and redness symptoms of the individual can be alleviated. In addition, the pharmaceutical composition of the present invention reduces the expression level of the inflammatory cytokine IL-6 in the individual and increases the expression level of the anti-inflammatory cytokine IL-10. Therefore, the pharmaceutical composition according to the present invention can be useful for preventing or treating rheumatoid arthritis.

### BRIEF DESCRIPTION OF THE DRAWINGS

FIG. 1 is a photograph that is taken to compare, with the naked eye, swelling and redness symptoms in mice feet of a normal group, a control group, or an experimental group administered with exogenous mitochondria.
FIG. 2 is a graph showing by scoring the degree of the disease symptoms of the mice in a normal group, a control group, or an experimental group administered with exogenous mitochondria.
FIG. 3 is a graph showing the measurement of the paw thickness in the dorsal axis of the mice in a normal group, a control group, or an experimental group administered with exogenous mitochondria.
FIG. 4 is a graph comparing IL-6 expression levels of the mice in a normal group, a control group, or an experimental group administered with exogenous mitochondria.
FIG. 5 is a graph comparing IL-10 expression levels of the mice in a normal group, a control group, or an experimental group administered with exogenous mitochondria.
FIG. 6 is a photograph obtained by photographing a distribution state of exogenous mitochondria in the body of rheumatoid arthritis-induced mice administered with the exogenous mitochondria.
FIG. 7 is a photograph obtained by photographing a histological state for evaluating the severity of rheumatoid arthritis according to the administration of exogenous mitochondria.
FIG. 8 is a graph showing that macrophages were proliferated by the administration of exogenous mitochondria even in the presence of LPS.
FIG. 9 is a graph showing that the secretion level of TNF-α was decreased by the administration of exogenous mitochondria even in the presence of LPS.
FIG. 10 is a graph showing that the secretion level of IL-6 was decreased by the administration of exogenous mitochondria even in the presence of LPS.

### BEST MODE FOR CARRYING OUT THE INVENTION

Hereinafter, the present invention will be described in detail.

One aspect of the present invention provides a pharmaceutical composition for preventing or treating rheumatoid arthritis, containing mitochondria as an active ingredient.

Unless otherwise particularly stated herein, the term "active ingredient" refers to an ingredient that exhibits activity alone or in combination with an adjuvant (carrier) having no activity by itself.

The mitochondria may be obtained from a mammal or from a human. Specifically, the mitochondria may be isolated from cells or tissues. For example, the mitochondria may be obtained from somatic cells, germ cells, or stem cells. In addition, the mitochondria may be normal mitochondria obtained from cells of which mitochondrial biological activity is normal. In addition, the mitochondria may be cultured *in vitro.*

Also, the mitochondria may be obtained autologously, allogeneically, or xenogeneically. Specifically, the term "autologous mitochondria" refers to mitochondria obtained from tissues or cells of the same individual. Also, the term "allogeneic mitochondria" refers to mitochondria obtained from an individual which belongs to the same species as an individual to which the mitochondria are to be applied but has a different genotype therefrom for alleles. In addition, the term "xenogenic mitochondria" refers to mitochondria obtained from an individual which belongs to a different species from an individual to which the mitochondria are to be applied.

Specifically, the somatic cells may be muscle cells, hepatocytes, neurons, fibroblasts, epithelial cells, adipocytes, osteocytes, leukocytes, lymphocytes, platelets, or mucosal cells.

In addition, the germ cells are cells undergoing meiosis and mitosis, and may be sperms or eggs. The stem cells may be any one selected from the group consisting of mesenchymal stem cells, adult stem cells, induced pluripotent stem cells, embryonic stem cells, bone marrow stem cells, neural stem cells, limbal stem cells, and tissue-derived stem cells. The mesenchymal stem cells may be any one selected from the group consisting of umbilical cord, cord blood, bone marrow, fat, muscle, nerve, skin, amnion, and placenta.

Meanwhile, in the case of isolating the mitochondria from a specific cell, the mitochondria may be isolated by various known methods such as using a specific buffer solution or using a potential difference and a magnetic field.

The isolation of mitochondria may be achieved by disrupting and centrifuging cells in consideration of maintaining the mitochondrial activity. In an embodiment, the isolation of mitochondria may be performed by: culturing cells and performing a first centrifugation on a pharmaceutical composition including the cultured cells to produce a pellet; resuspending the pellet in a buffer solution and homogenizing the pellet; performing a second centrifugation on the homogenized solution to produce a supernatant; and performing a third centrifugation on the supernatant to purify mitochondria. In this case, it is preferable to adjust the time taken to perform the second centrifugation to be shorter than the time taken to perform the first and third centrifugations in consideration of maintaining the cell activity, and the speed may be increased from the first centrifugation to the third centrifugation.

Specifically, the first to third centrifugation may be performed at a temperature of 0 to 10 °C, preferably 3 to 5 °C. In addition, the centrifugation may be performed for 1 to 50 minutes, and the centrifugation time may be appropriately adjusted according to the number of centrifugations, sample content, etc.

In addition, the first centrifugation may be performed at a speed of 100 to 1,000×g, 200 to 700×g, or 300 to 450×g. In addition, the second centrifugation may be performed at a speed of 1 to 2,000×g, 25 to 1,800×g, or 500 to 1,600×g. In addition, the third centrifugation may be performed at a speed of 100 to 20,000×g, 500 to 18,000×g, or 800 to 15,000×g.

In addition, mitochondria may be included in the pharmaceutical composition at a concentration of 0.1-500 pg/ml, 0.2-450 pg/ml or 0.5-400 pg/ml. By including mitochondria within the above range, the amount of mitochondria may be easily controlled during administration, and the symptoms of rheumatoid arthritis of a patient may be further improved.

In particular, the pharmaceutical composition according to the present invention may be administered with mitochondria in an amount of 0.01-5 mg/kg, 0.1-4 mg/kg, or 0.25-2.5 mg/kg per one time on the basis of the body weight of an individual to be administered. That is, it is most preferable in terms of the cell activity to administer the pharmaceutical composition such that the amount of mitochondria falls within the above range on the basis of the body weight of an individual in which rheumatoid arthritis is induced. In addition, the pharmaceutical composition may be administered 1-10 times, 3-8 times, or 5-6 times, and preferably 5 times. In this case, the administration interval may be 1-7 days, or 2-5 days, and preferably 3 days.

In addition, the pharmaceutical composition according to the present invention may be administered to a human or other mammal that is susceptible to rheumatoid arthritis or suffering from a disease or disorder associated with rheumatoid arthritis. In addition, the pharmaceutical composition may be an injectable preparation that may be intravenously administered, and may be preferably a preparation for injections.

Therefore, the pharmaceutical composition according to the present invention may be prepared as a physically or chemically highly stable injectable preparation by adjusting the pH of the composition by means of a buffer solution such as an acid aqueous solution or phosphate which may be used in an injectable preparation, in order to ensure the stability of the product during distribution of injectable preparations.

Specifically, the pharmaceutical composition of the present invention may contain water for injection. The water for injection is distilled water prepared for dissolving a solid injectable preparation or diluting a water-soluble injectable preparation, and may be glucose injection, xylitol injection, D-mannitol injection, fructose injection, saline, dextran 40 injection, dextran 70 injection, amino acid injection, Ringer's solution, lactic acid-Ringer's solution, phosphate buffer solution having a pH of 3.5 to 7.5, sodium dihydrogen phosphate-citrate buffer solution or the like.

In addition, the pharmaceutical composition of the present invention may include a stabilizer or a dissolution aid. For example, the stabilizer may be sodium pyrosulfite or ethylenediaminetetraacetic acid, and the dissolution aid may be hydrochloric acid, acetic acid, sodium hydroxide, sodium hydrogen carbonate, sodium carbonate or potassium hydroxide.

In addition, the present invention may provide a method for preventing or treating rheumatoid arthritis including administering the above-mentioned pharmaceutical composition to an individual. Here, the individual may be a mammal, and preferably a human.

In this case, the administration may be performed by intravenous administration. Therefore, the pharmaceutical composition according to the present invention may supply exogenous mitochondria having normal activity to the veins of an individual suffering from rheumatoid arthritis, thereby being useful for increasing the activity of cells having a lowered mitochondrial function, regenerating cells having a mitochondrial dysfunction, and being used for preventing or treating rheumatoid arthritis.

### MODE FOR THE INVENTION

Hereinafter, a preferred embodiment will be presented to help the understanding of the present invention. However, the following examples are provided only to easily understand the present invention, and the present invention is not limited to the following examples.

### Example 1. Preparation of Composition Containing Mitochondria

Umbilical cord-derived mesenchymal stem cells, which were provided from the CHA Bundang Medical Center, were seeded onto an Alpha-Minimum Essential Medium (Alpha-MEM) containing 10% (v/v) Fetal bovine serum (FBS, Gibco), 100 pg/ml of streptomycin, and 100 U/ml of ampicillin, and cultured for 72 hours. After the culture was completed, the cells were washed twice with Dulbecco's phosphate buffered saline (DPBS, Gibco). The washed cells were treated with 0.25% (v/v) trypsin-EDTA (TE, Gibco) to obtain cells.

In order to extract mitochondria, the obtained cells were harvested at a concentration of 1×10⁷ cells/ml using a hemocytometer. The cell line was subjected to a first centrifugation at a speed of 350×g for 10 minutes at a temperature of about 4 °C. The obtained pellet was collected, resuspended in a buffer solution, and homogenized for 10 to 15 minutes. Then, the composition containing the pellet was subjected to a second centrifugation at a speed of 1,100×g for three minutes at a temperature of about 4 °C, thereby obtaining a supernatant. The supernatant was subjected to a third centrifugation at a speed of 12,000×g for 15 minutes at a temperature of about 4 °C to isolate mitochondria from the cell line. The thus obtained mitochondria in an amount of 5 µg or 50 µg were mixed with 100 µl of water for injection, respectively, and then filled into an insulin syringe.

### Preparation Example 1. Preparation of Composition Containing Mesenchymal Stem Cells

Umbilical cord-derived mesenchymal stem cells, which were provided from the CHA Bundang Medical Center, were seeded onto an Alpha-MEM containing 10% (v/v) FBS, 100 pg/ml of streptomycin, and 100 U/ml of ampicillin, and cultured for 72 hours. After the culture was completed, the cells were washed twice with DPBS. The washed cells were treated with 0.25% (v/v) Trypsin-EDTA to obtain cells. The obtained cells were washed twice again with DPBS, mixed with 100 µl of water for injection in an amount of 1×10⁶ cells, and then filled into an insulin syringe.

### Preparation Example 2. Preparation of Composition Containing Tumor Necrosis Factor Inhibitor

The tumor necrosis factor inhibitor, Enbrel (etanercept), which was provided from the CHA Bundang Medical Center, was diluted to be administered in a dose of 8.3 µg based on 20 g of mouse weight, mixed with 100 µl of water for injection, and then filled into an insulin syringe. This dose corresponds to a dose of 25 mg based on 60 kg of the human body weight as calculated in an amount to be administered to a human.

### Experimental Example 1. Preparation of Rheumatoid Arthritis-Induced Mice and Administration of Composition

Six to eight week-old female DBA-1/j mice were purchased from Orient Bio Co., Ltd. (Seoul, Korea). The purchased mice were acclimatized in the clean zone of the Laboratory Animal Center of CHA university, and then the experiment was conducted. During the acclimation period, the environment in which the mice live was maintained at a 12-h light/dark cycle, a room temperature of 23±2 °C, and a humidity of 40 to 60%. After 7 days of such acclimation period, the mice were subjected to the experiments.

In order to induce rheumatoid arthritis in the mice subjected to the acclimation period, 4 mg/ml of chicken-derived type 2 collagen was dissolved in 0.05-0.1 M of acetic acid at a temperature of 4 °C, and the same amounts of complete Freund's adjuvant (CFA) and incomplete Freund's adjuvant (IFA) were mixed respectively to prepare a primary immune-inducing suspension (CFA) and a secondary immune-inducing suspension (IFA). 0.1 ml of the prepared primary immune-inducing suspension (CFA) was administered subcutaneously to the mice at the base of the tail, and after three weeks, 0.1 ml of the secondary immune-induced suspension (IFA) was further administered subcutaneously to the mice at the base of the tail to produce animal models of rheumatoid arthritis.

Then, 100 µl of the Enbrel-containing composition prepared in Preparation Example 2 was administered to rheumatoid arthritis-induced mice through a subcutaneous injection (SC) at three-day interval in total five times to thereby prepare Experimental Group 1. In addition, the composition containing the umbilical cord-derived mesenchymal stem cells as prepared in Preparation Example 1, and the composition containing the mitochondria as prepared in Example 1, were administered in an amount of 100 µl through an intravenous injection (IV) at three-day interval in total five times to thereby prepare Experimental Groups 2 to 4. In addition, normal mice that were not subjected to administration were prepared as a normal group. In addition, a control group was prepared in the same manner as in Experimental Group 2 except that 100 µl of water for injection was administered through an intravenous injection (IV) at three-day interval in total five times (Table 1).

**[Table 1]**

| | Normal Group | Control Group | Exp. Group 1 | Exp. Group 2 | Exp. Group 3 | Exp. Group 4 |
|---|---|---|---|---|---|---|
| Administered Composition | - | Water for injection | Prep. Ex. 2. (Enbrel) | Prep. Ex. 1. (MSCs) | Ex. 1. (MT-Low) | Ex. 1. (MT-High) |
| Dose | - | 100 µL | 25 mg/ 60 kg | 1 x 10⁶ cells | 5 µg | 50 µg |
| Number of mice | 7 | 7 | 8 | 8 | 8 | 8 |

Meanwhile, Enbrel is administered twice a week at a dose of 25 mg per one time with respect to an adult, and generally administered twice a week at a dose of 25 mg in total five times according to dosage and administration schedule in the case of an animal experiment using Enbrel.

### Experimental Example 2. Confirmation of Reduction in Swelling and Redness According to Administration of Exogenous Mitochondria

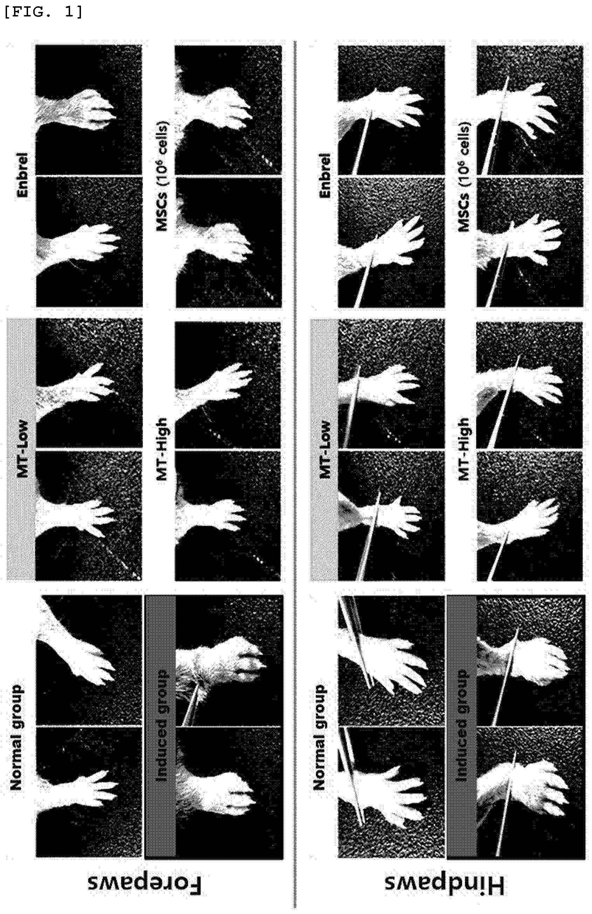

In order to evaluate visually observed symptoms after 30 days in each experimental group prepared in Experimental Example 1, the paws of the mice in a normal group, a control group, and experimental groups 1 to 4 were photographed and shown in FIG. 1.

As shown in FIG. 1, no swelling and redness were observed in the paws of the mice in the normal group. On the other hand, large and small joints were severely swollen in the paws of the mice in the control group, and the symptoms of redness were observed overall. In the paws of the mice in experimental groups 1 and 2 administered with Enbrel or mesenchymal stem cells, large and small joints were much swollen in the forepaws, and the symptoms of redness were observed overall. The swelling of the hindpaws of the mice in experimental groups 1 and 2 was relatively good, and the symptoms of redness were partially observed. In the paws of the mice in experimental groups 3 and 4 which were administered with mitochondria, the swelling of the forepaws and the hindpaws was relatively good, and the symptoms of redness were partially observed. Accordingly, it was confirmed with the naked eyes that the composition containing mitochondria has more excellent effects in reducing swelling and redness than Enbrel which is a tumor necrosis factor inhibitor.

### Experimental Example 3. Confirmation of Effects of Improving Symptoms of Rheumatoid Arthritis According to Administration of Exogenous Mitochondria

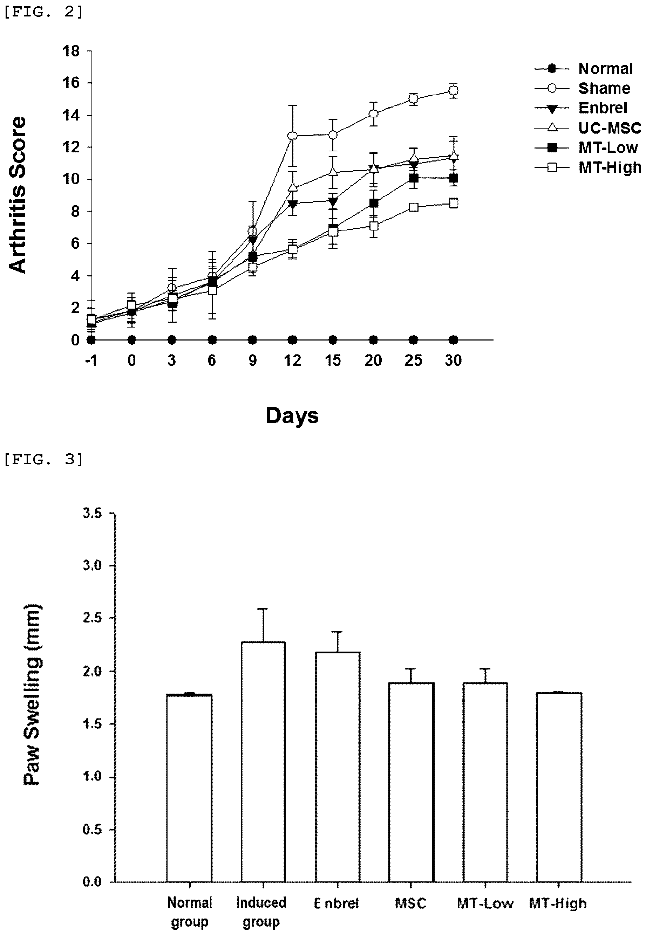

The degree of rheumatoid arthritis symptoms after 30 days was scored in each experimental group in Experimental Example 1 based on a score reference table (Table 2), and the results are shown in FIG. 2.

**[Table 2]**

| Score | Symptoms |
|---|---|
| 0 | No swelling or redness in small or large joints |
| 1 | Mild swelling and/or redness in small or large joints |
| 2 | Moderate swelling and/or redness in one or several small or large joints |
| 3 | Severe swelling and redness in large joints, and moderate swelling |
| | and/or redness in small joints |
| 4 | Very severe swelling and redness in large joints, and severe swelling and/or redness in small joints |

As shown in FIG. 2, no swelling or redness was shown in the paws of the mice in the normal group, and scores according to disease symptoms were determined as 0 points. On the other hand, very severe swelling and redness occurred in the large and small joints of the mice in the control group, and the scores according to disease symptoms were determined as 15 to 16 points. In addition, in the paws of the mice in the experimental group 1 and experimental group 2 administered with Enbrel or mesenchymal stem cells, severe swelling and redness occurred in the large joints, moderate swelling and redness occurred in the small joints, and the scores according to disease symptoms were determined as 11 to 12 points. On the contrary, moderate swelling and/or redness occurred in the large joints or the small joints of the mice in the experimental group 3 and experimental group 4 administered with mitochondria, and the scores according to disease symptoms were determined as 8 to 10 points. Accordingly, it was confirmed that the composition containing mitochondria has better therapeutic effect than Enbrel which is a tumor necrosis factor inhibitor.

### Experimental Example 4. Confirmation of Effects of Reducing Swelling According to Administration of Exogenous Mitochondria

In order to quantify the degree of swelling of the paw after 30 days in each experimental group prepared in Experimental Example 1, the paw thickness in the dorsal axis was analyzed by using a vernier caliper and is shown in FIG. 3. Here, the paw thicknesses of the forepaws and hindpaws in the dorsal axis were measured and averaged, and the results thereof were shown in a graph.

As shown in FIG. 3, the paw thickness in the dorsal axis of the mice in the control group was determined to be about 28% greater than that of the mice in the normal group. In addition, the paw thickness in the dorsal axis of the mice in the experimental group 1 administered with Enbrel was determined to be about 23% greater than that of the mice in the normal group. The paw thickness in the dorsal axis of the mice in the experimental group 2 administered with mesenchymal stem cells was determined to be about 6.8% greater than that of the mice in the normal group. On the contrary, the paw thicknesses in the dorsal axis of the mice in the experimental groups 3 and 4 administered with mitochondria were determined to be about 0.7 to 6% greater than that of the mice in the normal group. Therefore, it was confirmed that swelling was reduced in the experimental groups administered with the composition containing mitochondria.

### Experimental Example 5. Confirmation of Changes in Cytokine Expression Level According to Administration of Exogenous Mitochondria

In order to evaluate inflammatory cytokines present in the serum of the mice in the normal group, the control group, and the experimental groups 1 to 4 prepared in Experimental Example 1, 0.4 ml of blood was withdrawn from the mouse heart, and was then coagulated at room temperature for 20-30 minutes. The coagulated blood was centrifuged at 2,500 to 3,000 rpm to separate the supernatant serum. The separated serum was analyzed to check the expression levels of the inflammatory cytokine IL-6 and the anti-inflammatory cytokine IL-10 through enzyme-linked immunosorbent assay (ELISA).

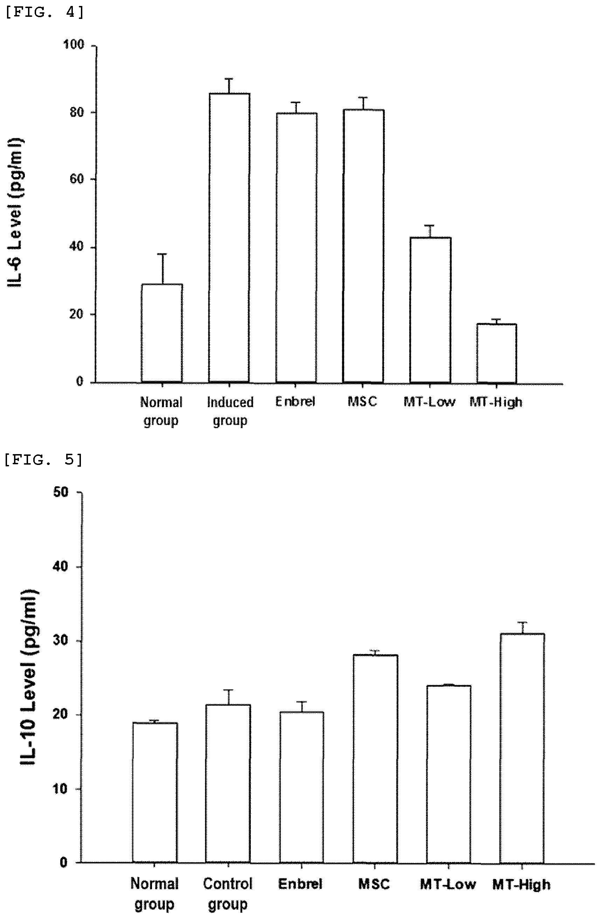

As shown in FIG. 4, the IL-6 expression level of the mice in the normal group was determined to be about 29 pg/ml. On the other hand, the IL-6 expression level of the mice in the control group was about 85 pg/ml, which was about 2.9 times higher than that of the normal group. In addition, the IL-6 expression levels of the mice in the experimental group 1 administered with Enbrel and the mice in the experimental group 2 administered with mesenchymal stem cells were determined to be about 79 pg/ml and about 81 pg/ml, respectively, which were similar to that of the control group. On the contrary, the IL-6 expression levels of the mice in the experimental groups 3 and 4 administered with the composition including mitochondria were determined to be about 42 pg/ml and about 17 pg/ml, respectively, which were similar to that of the normal group or became lower by about two times than that of the control group. This demonstrated that the composition containing mitochondria reduces the expression level of the inflammatory cytokine IL-6.

In addition, as shown in FIG. 5, the IL-10 expression level of the mice in the normal group was determined to be about 19 pg/ml. On the other hand, the IL-6 expression level of the mice in the control group was determined to be about 21 pg/ml. In addition, the IL-10 expression level of the mice in the experimental group 1 administered with Enbrel was about 20 pg/ml, which was similar to that of the control group. The IL-10 expression level of the mice in the experimental group 2 administered with mesenchymal stem cells was determined to be about 26 pg/ml, and was higher than those of the normal group and the control group. The IL-10 expression levels of the mice in the experimental groups 3 and 4 administered with the composition containing mitochondria were determined to be about 23 pg/ml and about 30 pg/ml, which were higher than those of the normal group and the control group. This demonstrated that the composition containing mitochondria increases the expression level of anti-inflammatory cytokine IL-10.

### Experimental Example 6. Confirmation of In-vivo Distribution in Rheumatoid Arthritis-induced Mice After Administration of Mitochondria

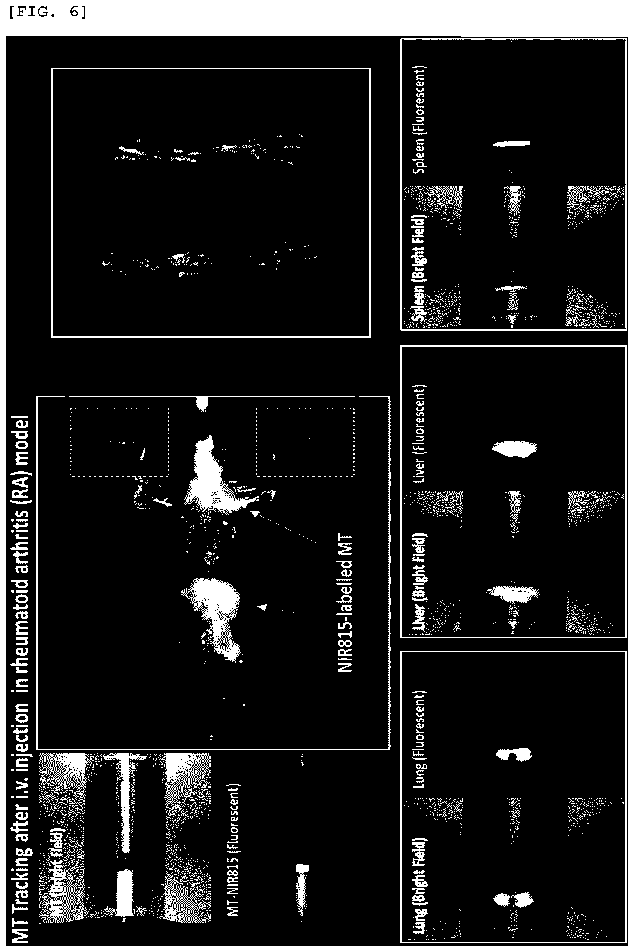

In order to confirm the *in vivo* distribution of mitochondria administered in rheumatoid arthritis-induced mice, mitochondria were labeled with a near-infrared fluorescent reagent, CellVue™ NIR815 fluorescent dye (Thermo Fisher Scientific, USA), and then administered via tail vein of the mice. In order to observe mitochondrial distribution after 3 hours, mitochondrial *in-vivo* distribution was photographed using an Impulse near-infrared equipment, and the photographed result is shown in FIG. 6.

As shown in FIG. 6, it was observed that mitochondria administered through the tail vein of the mice were distributed in the liver, lungs, and spleen along the vein blood vessels, and specifically present in the paws of the mice.

### Experimental Example 7. Confirmation of Improvement in Rheumatoid Arthritis Severity According to Administration of Exogenous Mitochondria Through Histological Evaluation

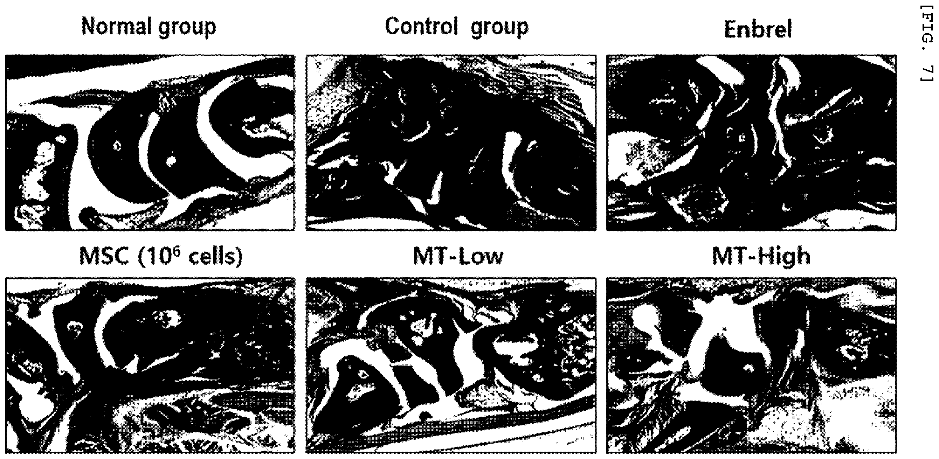

In order to analyze the severity of rheumatoid arthritis after 30 days in each experimental group prepared in Experimental Example 1, the paws of the mice were removed and fixed in 4% paraformaldehyde for two days. The fixed paw tissues were decalcified in the decalcifying solution lite (Sigma) to prepare a paraffin block. The prepared paraffin blocks of the paw tissues were cut into 10 µm slices and fixed. Then, the slices were stained with hematoxylin and eosin, and photographed through a microscope, and the results are shown in FIG. 7.

As shown in FIG. 7, the joint morphology was maintained in the paw tissues of the mice in the normal group, and no intra-articular infiltration of synovial fibroblasts caused by inflammatory responses was observed. On the other hand, intra-articular infiltration of inflammatory cells and synovial fibroblasts caused by inflammatory responses occurred in the paw tissues of the mice in the control group, thereby damaging the joint morphology. In addition, intra-articular infiltration of inflammatory cells and synovial fibroblasts, which was similar as in the control group, was observed in the paw tissues of the mice in the experimental group 1 administered with Enbrel and the experimental group 2 administered with mesenchymal stem cells.

On the other hand, intra-articular infiltration of inflammatory cells and synovial fibroblasts was reduced in the paw tissues of the mice in the experimental groups 3 and 4 administered with the composition containing mitochondria and the joint morphology was similar to that of the normal group. This demonstrated that the composition containing mitochondria inhibits inflammatory cell infiltration and abnormal proliferation of synovial fibroblasts through inflammatory responses.

### Experimental Example 8. Confirmation of Macrophage Proliferation by Mitochondrial Transfer

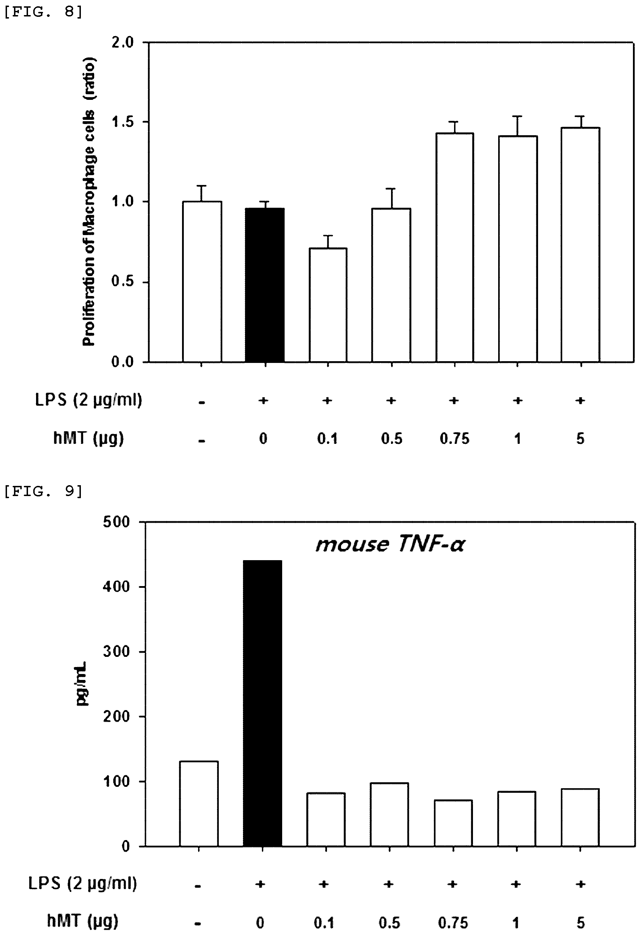

Mouse-derived Raw 264.7 (macrophage) cells were treated with LPS (2 pg/ml; L4391, Sigma) for 6 hours to induce an inflammatory response. Then, healthy mitochondria extracted from human UC-MSCs were transferred thereto at different concentrations (0.1, 0.5, 0.75, 1, 5 µg), and seeded onto a 24-well plate to be incubated in a CO₂ incubator at 37 °C. After 24 hours have elapsed, the supernatants of the samples were collected and centrifuged at 1,500 rpm for 5 minutes, and only the supernatants were transferred to new tubes. In order to compare cell proliferation ability, EZ-cytoX kit (EZ-3000, Dogen) was used. A reaction solution contained in a test kit was mixed with each of the obtained samples at a ratio of 10:1 (reaction solution (vol.)=1/10 of the sample), then reacted for 2 hours in a CO₂ incubator at 37 °C, and thereafter the absorbance at 450 nm was measured. As a result, as shown in FIG. 8, mitochondrial dehydrogenase activity increased as the number of viable cells in the samples increased.

### Experimental Example 9. Confirmation of Inhibitory Efficacy of Pro-inflammatory Cytokine Secretion of Macrophages by Mitochondrial Transfer

In the same manner as in Experimental Example 8, mouse-derived Raw 264.7 (macrophage) cells were treated with LPS (2 pg/ml) for 6 hours to induce an inflammatory response. Then, healthy mitochondria extracted from human UC-MSCs were transferred thereto at different concentrations (0.1, 0.5, 0.75, 1, 5 µg), and seeded onto a 24-well plate to be incubated in a CO₂ incubator at 37 °C. After 24 hours have elapsed, the supernatants of the samples were collected, centrifuged at 1,500 rpm for 5 minutes, and only the supernatants were transferred to new tubes. In order to confirm the expression levels of TNF-α which is a pro-inflammatory cytokine in macrophages and IL-6, the expression levels were analyzed using a mouse TNF-α ELISA kit (Sigma, #RAB0477) and a mouse IL-6 ELISA kit (Sigma, #RAB0308), respectively.

The supernatant sample for each condition was prepared by diluting the sample at a ratio of 1/10 using a dilution solution contained in an ELISA kit. The prepared sample and the standard solution each were put into 96-well plates on which the antibodies were pre-coated, and the plates were incubated overnight at 4 °C. On the next day, the solution remaining on the 96-well assay plate was removed using a washing solution contained in the assay kit. Subsequently, a biotin-labeled detection antibody as prepared was added to each well and reacted for 1 hour at room temperature. After the reaction was completed, the solution remaining on the 96-well assay plate was removed using the washing solution. Next, a streptavidin solution as prepared was added to each well, reacted at room temperature for 45 minutes using a stirrer, and then the solution remaining on the 96-well assay plate was removed using the washing solution. Finally, a substrate reagent solution was added thereto and reacted for 30 minutes using a stirrer at room temperature and under the light-shielding condition. After 30 minutes of reaction, a stop solution was added to each well and the absorbance at 450 nm was measured.

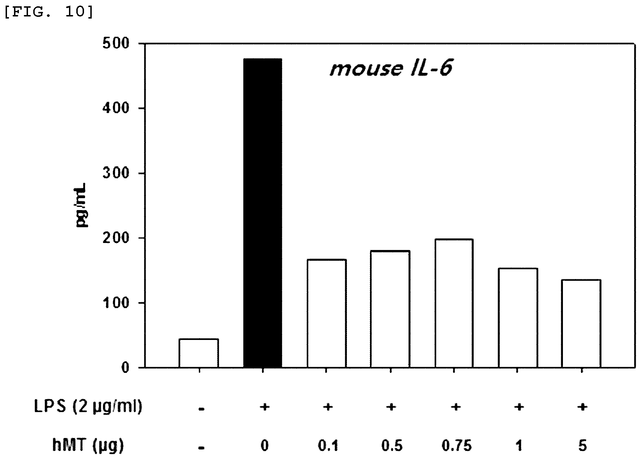

As a result, as shown in FIGS. 9 and 10, it was confirmed that, although the cells were treated with LPS, the expression levels of TNF-α and IL-6 were effectively reduced by mitochondrial treatment.

## Claims

1. A pharmaceutical composition for preventing or treating rheumatoid arthritis, the composition comprising mitochondria as an active ingredient.

2. The pharmaceutical composition of claim 1, wherein the mitochondria are isolated from cells or tissues.

3. The pharmaceutical composition of claim 2, wherein the cells are any one selected from the group consisting of somatic cells, germ cells, stem cells and a combination thereof.

4. The pharmaceutical composition of claim 3, wherein the somatic cells are any one selected from the group consisting of muscle cells, hepatocytes, neurons, fibroblasts, epithelial cells, adipocytes, osteocytes, leukocytes, lymphocytes, platelets, mucosal cells, and a combination thereof.

5. The pharmaceutical composition of claim 3, wherein the germ cells are any one selected from the group consisting of sperms, eggs, and a combination thereof.

6. The pharmaceutical composition of claim 3, wherein the stem cells are any one selected from the group consisting of mesenchymal stem cells, adult stem cells, induced pluripotent stem cells, embryonic stem cells, bone marrow stem cells, neural stem cells, limbal stem cells, tissue-derived stem cells, and a combination thereof.

7. The pharmaceutical composition of claim 6, wherein the mesenchymal stem cells are any one selected from the group consisting of umbilical cord, cord blood, bone marrow, fat, muscle, nerve, skin, amnion, placenta, synovial fluid, testis, periosteum, and a combination thereof.

8. The pharmaceutical composition of claim 1, wherein the mitochondria are included at a concentration of 0.1-500 pg/ml with respect to the pharmaceutical composition.

9. A method for preventing or treating rheumatoid arthritis, the method comprising administering the pharmaceutical composition of any one of claims 1-8 to an individual.
